Europäisches Patentamt

European Patent Office

Office européen des brevets

(11) Veröffentlichungsnummer: **0 481 241 A1**

## EUROPÄISCHE PATENTANMELDUNG

(21) Anmeldenummer: **91116026.5**

(22) Anmeldetag: **20.09.91**

(51) Int. Cl.5: **A61G 13/10, A61B 19/08**

(30) Priorität: **19.10.90 DE 4033243**

(43) Veröffentlichungstag der Anmeldung:
**22.04.92 Patentblatt 92/17**

(84) Benannte Vertragsstaaten:
**BE ES FR GB IT LU NL**

(71) Anmelder: **GFM MASCHINENBAU GmbH**
**22, Lohausstrasse**
**W-4358 Haltern(DE)**

(72) Erfinder: **Gawarecki, Herbert, Ing. grad.**
**12, Albert-Schweitzer-Strasse**
**W-4358 Haltern(DE)**

(74) Vertreter: **Eichelbaum, Lambert, Dipl.-Ing.**
**Krüppeleichen 6**
**W-4350 Recklinghausen(DE)**

(54) **Operationstischbezug.**

(57) Die Erfindung betrifft einen Operationstischbezug (1), bestehend aus einem Folienschlauch (2) von thermoplastischem Kunststoff mit einem geschlossenen Bodenbereich (3) und einem offenen Kopfbereich (4), wobei der Folienschlauch (2) auf seiner Oberseite (5) mit einem saugfähigen Faservlies (6) belegt, quer zur Längsrichtung umgefaltet und der Bodenbereich (3) auf den Kopfbereich (4) gelegt ist und letzterer (4) mit einem umlaufenden Stulpenrand den Bodenbereich (3) übergreift.

Der Erfindung liegt die Aufgabe zugrunde, einen Operationstischbezug (1) dieser Gattung zu schaffen, der unter Beibehaltung seines Falt- und Stulpenprinzips rascher und mit geringerer Kraftanstrengung über den Operationstisch (9) gestreift werden kann.

Diese Aufgabe wird erfindungsgemäß dadurch gelöst, daß der Stulpenrand (12, 21) die gleiche Breite wie der Folienschlauch (2) aufweist und mit seinem freien Ende (25) zu einem mindestens zwei Lagen (17, 20, 22, 23) umfassenden Stulpenrand (12, 21) mit der außenliegenden Lage (17) in Richtung auf die Öffnung (14) im Kopfbereich (4) umfaltet ist.

Fig.2

Die Erfindung betrifft einen Operationstischbezug, bestehend aus einem Folienschlauch von thermoplastischem Kunststoff mit einem geschlossenen Bodenbereich und einem offenen Kopfbereich, wobei der Folienschlauch auf seiner Oberseite mit einem saugfähigen Faservlies belegt, quer zur Längsrichtung umgefaltet und der Bodenbereich auf den Kopfbereich gelegt ist sowie letzterer mit einem umlaufenden Stulpenrand den Bodenbereich übergreift.

Ein Operationstischbezug dieser Art ist aus der DE-OS 37 15 691 bekannt geworden. Derartige Operationstischbezüge werden von einer Operationsschwester an beiden Seitenbereichen mit den Händen innerhalb des umlaufenden Stulpenrandes ergriffen, auseinandergezogen und vom freien Ende des Operationstisches derart übergezogen, daß das saugfähige Faservlies sowie der auf den Kopfbereich gefaltete Bodenbereich auf der Oberseite des Operationstisches angeordnet sind. Dabei kann die vom Körper der Bedienungsperson hinweg erfolgende Schubbewegung des Tischbezuges in Richtung auf den Operationstisch bis zu dessen umgefalteten Mittenrand relativ rasch durchgeführt werden. Hiernach erfolgt jedoch die weitere Überzugs- bzw. Schubbewegung wegen der an der Knickstelle des um 180° abrollenden Tischbezuges entgegen einer wesentlich höheren Reibkraft und daher mit einer erhöhten Kraftanstrengung.

Danach muß der Stulpenrand nach dem vollständigen Aufzug des Bezuges durch seitliches Auseinanderdrücken mit den Handaußenflächen in Richtung auf die Öffnung im Kopfbereich umgestülpt werden, wobei er einreißen kann. Und schließlich sind die relativ harten Schweißnähte an den Längsseiten des Tischbezuges mit einer Verletzungsgefahr beim Entlangstreifen verbunden.

Von diesem Stand der Technik ausgehend, liegt der Erfindung die Aufgabe zugrunde, einen Operationstischbezug der eingangs genannten Gattung zu schaffen, der unter Beibehaltung seines Falt- und Stulpenprinzips rascher und mit geringerer Kraftanstrengung über den Operationstisch gestreift werden kann.

Diese Aufgabe wird in Verbindung mit dem eingangs genannten Gattungsbegriff erfindungsgemäß dadurch gelöst, daß der Stulpenrand die gleiche Breite wie der Folienschlauch aufweist und mit seinem freien Ende zu einem mindestens zwei Lagen umfassenden Stulpenrand mit der außenliegenden Lage in Richtung auf die Öffnung im Kopfbereich umfaltet ist.

Durch diese Ausbildung kann der Operationstischbezug wie bisher gehandhabt und von der Operationsschwester zunächst vom freien Ende des Operationstisches aus bis zu seinem umgefalteten Rand im Mittenbereich hin übergestreift werden. Sodann wird von der entgegengesetzten Seite die außenliegende Lage des Stulpenrandes zwischen Zeigefinger und Daumen beider Hände ergriffen und vollständig über den Rest des Operationstisches gezogen, wobei der mindestens zwei Lagen umfassende Stulpenrand auseinandergezogen wird. Dadurch entfällt ein kraftaufwendiges Umstülpen des beim Stand der Technik einlagigen Stulpenrandes und die damit verbundenen Einreißgefahren. Außerdem ist ein kontinuierliches Ziehen ergonomisch günstiger und schneller zu bewerkstelligen, wodurch sich die gesamte Handhabung des Operationstischbezuges bei seiner Aufbringung vereinfacht.

Nach einer besonders vorteilhaften Weiterbildung der Erfindung ist der Folienschlauch etwa in der Mitte des auf seiner Oberseite befindlichen Faservlieses gefaltet und der Stulpenrand aus vier am Kopfbereich umlaufenden Lagen gebildet. Durch die Faltung des Faservlieses in der vorbeschriebenen Weise wird es nochmals durch die Mittenfaltung steril durch sich selbst geschützt, während der nunmehr vierlagige Stulpenrand die bisherige Länge des Tischbezuges, die selbstverständlich vor Einführung in eine Verpakkung noch mehrfach gefaltet wird, kürzer gestaltet. Dementsprechend verkürzt sich auch die primäre Überstreiflänge, bevor der außenliegende Stulpenrand von der Bedienungsperson zwischen Daumen und Zeigefinger beider Hände ergriffen und über den Restbereich des Operationstisches gezogen wird.

Um vor dem Überstreifen sowohl das freie Ende der Öffnung für den Benutzer sichtbar werden zu lassen als auch nach dem Überzug bis zum quergefalteten Mittenbereich die außenliegende Lage des Stulpenrandes bequem sehen und rasch ergreifen zu können, ragt die innenliegende, die Öffnung im Kopfbereich bildende Lage nach einer vorteilhaften Weiterbildung der Erfindung ein wenig vor.

Vorteilhaft ist der Bodenbereich durch eine zur Längsrichtung des Folienschlauches querverlaufende Schweißnaht und der offene Kopfbereich durch einen zur Längsrichtung des Folienschlauches querverlaufenden Schnitt gebildet. Hingegen weist der Folienschlauch in seinen Seitenbereichen keine Naht auf. Dadurch ist die Schweißnaht auf den relativ kleinen Bodenbereich begrenzt und der Bezug ansonsten völlig glatt ausgebildet.

Ein Ausführungsbeispiel der Erfindung ist in den Zeichnungen dargestellt. Dabei zeigen:

Fig. 1    Die perspektivische Draufsicht auf einen Operationstisch mit vollständig übergestreiftem Bezug,

Fig. 2    eine Schnittansicht entlang der Linie II-II mit einem teilweise übergestreiften Bezug mit einem zweilagigen Stulpenrand,

Fig. 3    eine der Fig. 2 entsprechende Schnittdarstellung mit einem vierlagigen Stulpenrand und mittig umgefalteten Faservlies,

Fig. 4      eine perspektivische Schnittdarstellung durch einen Bezug mit einem zweilagigen Stulpenrand während dessen Überstreifen über einen Operationstisch,

Fig. 5      die perspektivische Draufsicht auf den Operationstischbezug in vollständig auseinandergezogener Länge und Breite und

Fig. 6      die Schnittansicht entlang der Linie VI-VI von Fig. 5.

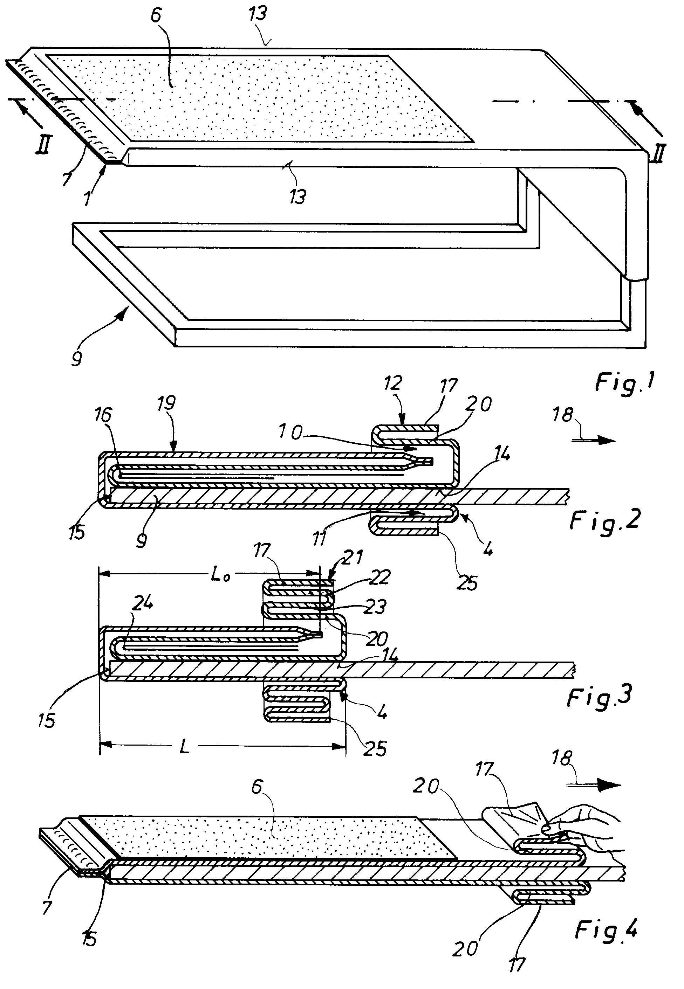

Der neue Operationstischbezug (1) gemäß den Figuren 1, 5 und 6 besteht aus einem Folienschlauch (2) von thermoplastischem Kunststoff mit einem geschlossenen Bodenbereich (3) und einem offenen Kopfbereich (4), wobei der Folienschlauch (2) auf seiner Oberseite (5) großflächig mit einem saugfähigen Faservlies (6) belegt ist. Der Folienschlauch (2) weist lediglich in seinem Bodenbereich (3) eine Schweißnaht (7) auf und ist in seinen sämtlichen übrigen Bereichen, insbesondere auch in seinen Seitenbereichen (8) nahtlos ausgebildet.

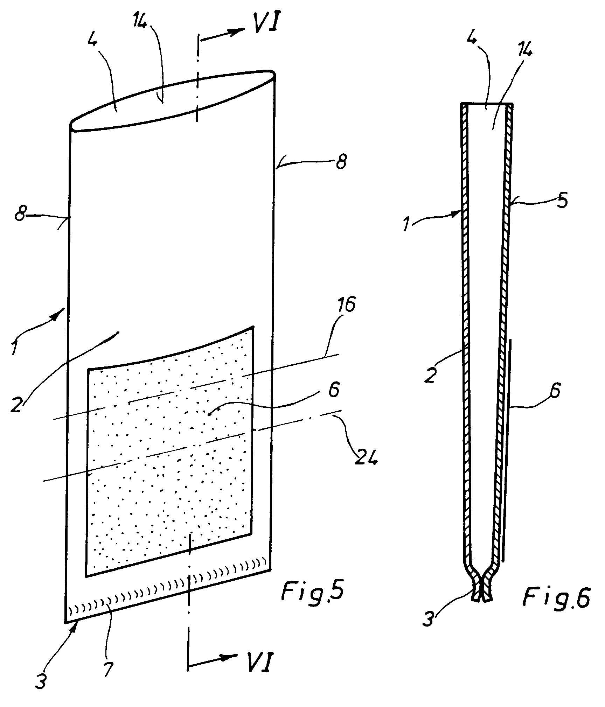

Fig. 1 zeigt den Operationstischbezug der Figuren 5 und 6 nach seinem vollständigen Überstreifen über einen Operationstisch (9).

Dieses Überstreifen erfolgt gemäß der Ausführungsform der Fig. 2 dadurch, daß die betreffende Bedienungsperson, z.B. eine Operationsschwester, vor dem Überstreifen mit ihren Händen in die Zwischenräume (10, 11) parallel zu beiden Längsseiten (13) des Operationstisches (9) in den Stulpenrand (12) greift und die Öffnung (14) im Kopfbereich (4) über die Kante (15) des Operationstisches (9) schiebt.

Bei dieser Ausführungsform ist das Faservlies (6) gemäß Fig. 5 etwa in Höhe der Faltlinie (16) umgefaltet. Sodann ergreift die Bedienungsperson von der entgegengesetzten Seite des Operationstisches (9), wie am anschaulichsten aus Fig. 4 ersichtlich ist, mit dem Daumen und dem Zeigefinger einer jeden Hand die außenliegende Lage (17) des Stulpenrandes (12) und zieht diesen (12) in Richtung des Pfeiles (18) von Fig. 2 und 4 auseinander. Dadurch wird einerseits der Stulpenrand (12) auseinandergezogen und andererseits der umgefaltete Teil (19) des vorliegenden Folienschlauches (2) über die Kante (15) des Operationstisches (9) abgerollt, bis die Naht (7) des Bodenbereiches (3) die in Fig. 1 dargestellte Lage eingenommen hat.

Um vor dem Überstreifen sowohl das freie Ende der Öffnung (14) für den Benutzer sichtbar werden zu lassen als auch nach dem primären Überstreifen bis zum quergefalteten Mittenbereich die außenliegende Lage (17) des Stulpenrandes (12) bequem sehen und rasch ergreifen zu können, ragt die innenliegende, die Öffnung (14) im Kopfbereich (4) bildende Lage (20) nach einer vorteilhaften Weiterbildung der Erfindung ein wenig vor.

Eine weitere Ausführungsform ist in Fig. 3 dargestellt. Bei dieser Ausführungsform besteht der Stulpenrand (21) aus insgesamt vier Lagen (17, 20, 22, 23). Dabei ist wie beim Ausführungsbeispiel der Fig. 2 die außenliegende Lage mit der Bezugsziffer (17) und die innenliegende Lage mit der Bezugsziffer (20) bezeichnet. Zwischen diesen befinden sich noch zwei weitere Lagen (22, 23), so daß bei dieser zweiten Ausführungsform der Stulpenrand (21) vierlagig ausgebildet ist. Zugleich wird bei dieser Ausführungsform das Faservlies (6) gemäß Fig. 5 exakt in seiner Mitte entlang der Faltlinie (24) gefaltet, wie deutlich aus Fig. 3 hervorgeht. Dadurch wird die sterile Oberfläche des Faservlieses (6) noch besser geschützt. Zugleich wird durch diese Ausführungsform die Gesamtlänge (L) gegenüber der entsprechenden Länge der Ausführungsform gemäß Fig. 2 verkürzt.

Dies ist mit dem handhabungstechnischen Vorteil verknüpft, daß die Bedienungsperson den von ihrem Körper fortführenden, primären Überstreifvorgang nur auf diese verkürzte Länge (L) beschränken muß. Hiernach kann die außenliegende Lage (17) des Stulpenrandes (21), wie in Fig. 4 dargestellt, mit dem Zeigefinger und dem Daumen beider Hände in der Nähe der Längsseiten (13) des Operationstisches (9) ergriffen und der Bezug (1) in einem sekundären Überstreifvorgang vollständig über den Operationstisch (9) gezogen werden. Da nunmehr auch die Länge (Lo) des umgefalteten und über die Tischkante (15) abzurollenden Bezugteiles verkürzt wird, ist der Bezug des Operationstisches (9) mit einem geringeren Kraftaufwand verbunden, zumal das Auseinanderziehen des Stulpenrandes (21) der Ausführungsform der Fig. 3 ergonomisch günstiger als das Abrollen der Abrollänge (Lo) über die Tischkante (15) ist.

Es versteht sich, daß die einzelnen Lagen (17, 20, 22, 23) des Stulpenrandes (12) bzw. (21) sowohl andere Faltlängen als auch weitere Umfaltungen zulassen, ohne daß dadurch der Erfindungsgedanke eingeschränkt wird. Erfindungswesentlich ist jedoch bei weiteren Ausgestaltungen, daß die Stirnseite (25) der jeweils außenliegenden Lage (17) des Stulpenrandes (12, 21) der Öffnung (14) des Kopfbereiches (4) zugekehrt ist.

Die Schnittdarstellungen gemäß den Figuren 2 bis 4 geben die tatsächlichen Dickenverhältnisse des Folienschlauches (2) im Interesse einer deutlichen Darstellung der Stulpenränder (12, 21) nicht wieder. Da der Folienschlauch (2) eine Dicke von lediglich 0,03 bis 0,2 aufweist, sind die Stulpenränder (12, 21) entgegen dem Stand der Technik nach der DE-OS 37 15 691 mit der gleichen Breite versehen. Sie weisen jedoch im Gegensatz zum vorbeschriebenen Stand der Technik keine zu verschweißenden Ein- oder Ausschnittkanten auf.

B e z u g s z e i c h e n l i s t e :

| | |
|---|---|
| Operationstischbezug | 1 |
| Folienschlauch | 2 |
| Bodenbereich | 3 |
| Kopfbereich | 4 |
| Oberseite des Folienschlauches 2 | 5 |
| Faservlies | 6 |
| Schweißnaht | 7 |
| Seitenbereich | 8 |
| Operationstisch | 9 |
| Zwischenräume | 10, 11 |
| Stulpenrand | 12, 21 |
| Längsseite des Operationstisches 9 | 13 |
| Öffnung im Kopfbereich 4 | 14 |
| Kante des Operationstisches 9 | 15 |
| Faltlinie | 16, 24 |

| | |
|---|---|
| Lagen der Stulpenränder 12, 21 | 17, 20, 22, 23 |
| Pfeil | 18 |
| umgefalteter Teil | 19 |
| Stirnseite | 25 |
| Abrollänge | Lo |
| Gesamtlänge | L |

**Patentansprüche**

1. Operationstischbezug, bestehend aus einem Folienschlauch von thermoplastischem Kunststoff mit einem geschlossenen Bodenbereich und einem offenen Kopfbereich, wobei der Folienschlauch auf seiner Oberseite mit einem saugfähigen Faservlies belegt, quer zur Längsrichtung umgefaltet und der Bodenbereich auf den Kopfbereich gelegt ist sowie letzterer mit einem umlaufenden Stulpenrand den Bodenbereich übergreift, **dadurch gekennzeichnet,** daß der Stulpenrand (12, 21) die gleiche Breite wie der Folienschlauch (2) aufweist und mit seinem freien Ende (25) zu einem mindestens zwei Lagen (17, 20, 22, 23) umfassenden Stulpenrand (12, 21) mit der außenliegenden Lage (17) in Richtung auf die Öffnung (14) im Kopfbereich (4) umgefaltet ist.

2. Operationstischbezug nach Anspruch 1, **dadurch gekennzeichnet,** daß der Folienschlauch (2) etwa in der Mitte des auf seiner Oberseite (5) befindlichen Faservlieses (6) gefaltet und der Stulpenrand (21) aus vier am Kopfbereich (4) umlaufenden Lagen (17, 20, 22, 23) gebildet ist.

3. Operationstischbezug nach Anspruch 1 oder 2, **dadurch gekennzeichnet,** daß die innenliegende, die Öffnung (14) im Kopfbereich (4) bildende Lage (20) des Stulpenrandes (12, 21) gegenüber der bzw. den außenliegenden Lagen (17, 22, 23) ein wenig hervorragt.

4. Operationstischbezug nach einem der Ansprüche 1 bis 3, **dadurch gekennzeichnet,** daß der Bodenbereich (3) durch eine zur Längsrichtung des Folienschlauches (2) querlaufende Schweißnaht (7) gebildet ist.

5. Operationstischbezug nach einem der Ansprüche 1 bis 4, **dadurch gekennzeichnet,** daß der offene Kopfbereich (4) durch einen zur Längsrichtung des Folienschlauches (2) querverlaufenden Schnitt gebildet ist.

6. Operationstischbezug nach einem der Ansprüche 1 bis 5, **dadurch gekennzeichnet,** daß der Folienschlauch (2) in seinen Seitenbereichen (8) nahtlos ausgebildet ist.

Fig.1

Fig.2

Fig.3

Fig.4

Fig.5

Fig.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | US-A-3 747 655 (HADTKE)<br>* das ganze Dokument *<br>--- | 1-5 | A61G13/10<br>A61B19/08 |
| A | DE-A-3 519 705 (SENGEWALD)<br>* Seite 9, Zeile 1 - Zeile 16; Abbildungen 1-4 *<br>--- | 2,3-6 | |
| A | US-A-3 335 719 (BOUCHER)<br>* Spalte 3, Zeile 16 - Zeile 45; Abbildungen 1-6 *<br>--- | 1 | |
| A | EP-A-0 290 738 (WEIMAR)<br>* das ganze Dokument * | 1 | |
| D | & DE-A-3 715 691 (WEIMAR)<br>--- | | |
| P,X | DE-U-9 014 501 (GFM MACCHINENBAU GMBH)<br>* das ganze Dokument *<br>----- | 1-6 | |

RECHERCHIERTE
SACHGEBIETE (Int. Cl.5 )

A61G
A61B

Der vorliegende Recherchenbericht wurde für alle Patentansprüche erstellt

| Recherchenort | Abschlußdatum der Recherche | Prüfer |
|---|---|---|
| DEN HAAG | 07 JANUAR 1992 | BAERT F. |